# EUROPEAN PATENT SPECIFICATION

(11) **EP 0 807 447 B1**
(45) Date of publication and mention of the grant of the patent: **04.12.2002**
(21) Application number: 97201316.3
(22) Date of filing: 02.05.1997
(51) Int. Cl.: A61M 25/01, A61M 25/00

(54) **Catheter-tip-straightener**
Katheterspritzenrichter
Redresseur de la pointe d'un cathéter

(30) Priority: 15.05.1996 NL 1003135
(43) Date of publication of application: 19.11.1997
(73) Proprietor: CORDIS EUROPA N.V., NL-9301 LJ Roden (NL)
(72) Inventor: Wenning, Hans Onno, 9725 LC Groningen (NL)
(74) Representative: 't Jong, Bastiaan Jacob

(56) References cited:
- EP-A- 0 328 760
- EP-A- 0 329 365
- WO-A-96/20024
- US-A- 3 853 130
- US-A- 4 585 437
- US-A- 5 282 479
- US-A- 5 320 613
- US-A- 5 484 419

## Description

The invention relates to a catheter-tip-straightener comprising a relatively stiff tubular body with a central channel.

Such a catheter-tip-straightener is used with catheters which have a curved tip, such as in particular catheters used for angiographic purposes. The curved tip is received inside the channel of the tip straightener, so that the tip is straightened and can easily be passed onto a guide wire. Subsequently the catheter is introduced into the body of the patient over the guide wire and through a catheter-introduction-sheath.

EP 0 329 365 describes a catheter grip for holding a catheter in a passage formed therein, and which facilitates insertion of the catheter in an introduction sheath.

The known catheter-tip-straightener may be a hindrance when introducing the catheter into the introduction sheath.

After inserting the guide wire into the catheter, the catheter-tip-straightener according to the invention as characterised in claim 1 may be connected to the catheter-introduction-sheath, so that it forms, as one whole, a continuation of it. Introduction of the catheter can in that case take place inside the tip straightener, which will provide a good grip.

Preferably the measure as set out in claim 2 is employed. The connecting means can be engaged with the introduction sheath in the somewhat compressed state of the tubular body and remain, due to the elastic action, firmly locked inside this introduction sheath. Additionally the added advantage is achieved that the catheter can be gripped tightly inside the tip straightener, so that it can be positioned properly inside it for the purpose of inserting the guide wire.

With a suitable embodiment the measure as set out in claim 3 is employed. The stop prevents the catheter-tip-straightener from being inserted too far into the introduction sheath, which could result in the membrane of the device being pushed open, possibly resulting in leakage. In a suitable manner the measure as set out in claim 4 is additionally employed. In that case the tip straightener will be positioned against the end of the introduction sheath over the entire circumference.

After introducing the first section of the catheter, the catheter-tip-straightener may be removed by taking the catheter out of the tip straightener via the longitudinal slit. Another suitable possibility is to move the tip straightener to the proximal end of the catheter onto the reinforcing sleeve usually arranged there. To this end the catheter according to the invention embodies preferably the measure as characterised in claim 5. Due to the funnel-shaped end, the relatively proximal end of the tip straightener can be pushed onto the reinforcing sleeve in a clamping manner, after which this tip straightener reinforces or takes over the action of the reinforcing sleeve. As a result, buckling of the catheter will be prevented effectively.

In order to be able to arrange the catheter-tip-straightener easily on the catheter, whilst at the same time is stays in position in a reliable manner, the measure as set out in claim 6 is preferable employed. The catheter can easily be inserted into the widened section of the longitudinal slit and advanced further into the channel.

In order to be able to manipulate the catheter-tip-straightener properly, both on insertion of the guide wire into the straightened tip received inside the tip straightener, and when the tip straightener functions as extension piece of the introduction sheath, the measure as set out in claim 7 is preferably employed. A suitable embodiment has additionally been characterised in claim 8, which does not affect the elastic compressibility of the tip straightener in an unfavourable manner.

The invention relates to and also provides an assembly for a catheter-introduction-sheath and a catheter-tip-straightener as characterised in claim 9.

The invention will be explained in greater detail in the following description with reference to the attached drawings.
- Figure 1: illustrates the catheter-tip-straightener according to the invention when in use.
- Figure 2: shows a partly cross-sectional view of an assembly of a catheter-introduction-sheath and a catheter-tip-straightener without a catheter.
- Figures 3 and 4: show two partly schematic views of an assembly as illustrated in figure 2 in a position of use.

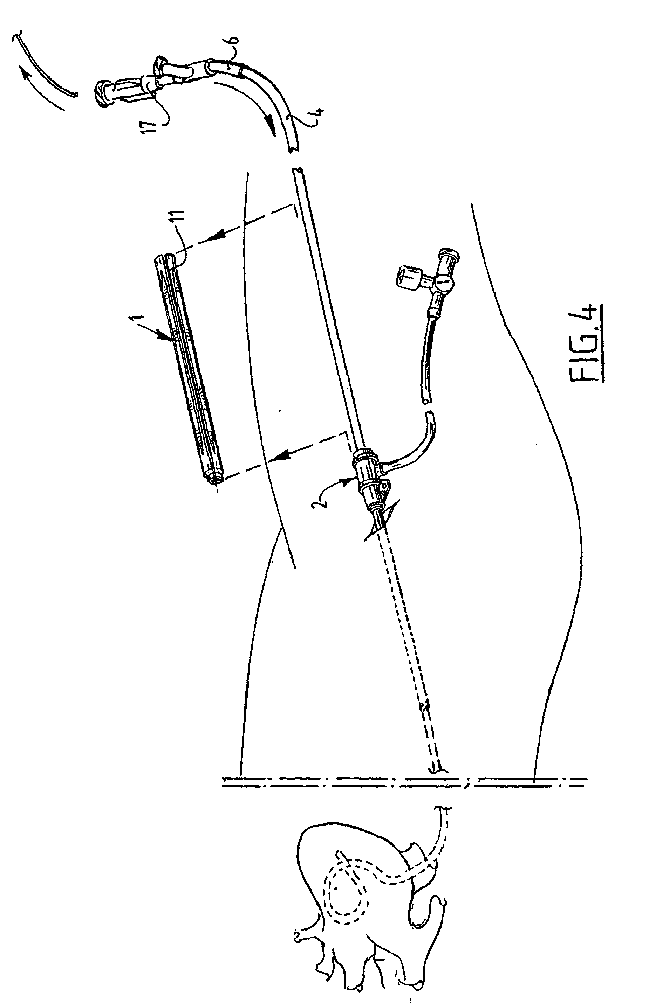

The catheter-tip-straightener 1 illustrated in figure 4 has been designed for the purpose of straightening the curved tip of a catheter 4, such as an angiography catheter, and to hold it whilst manipulating.

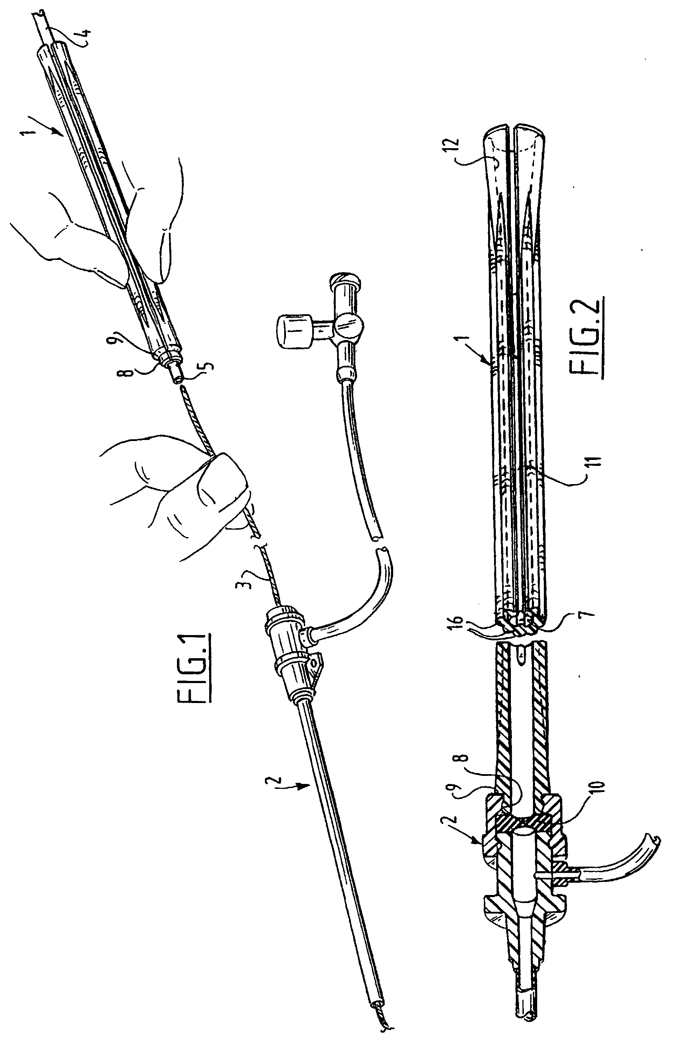

As can be seen in figure 1, the tip straightener 1 comprises a relatively stiff tubular body with a central channel 7 and a longitudinal slit 11 arranged in a wall of the body.

In order to introduce the catheter 4 into the body of the patient, a catheter-introduction-sheath 2 is employed in the usual manner. In figure 1 the latter has been illustrated whilst outside the body of a patient. First a guide wire 3 is advanced via the introduction sheath 2 to the site where the existing tip of the catheter is to finish up ultimately. The catheter 4 is introduced over this guide wire 3. To this end the guide wire 3 is passed into a guide wire 5 of the catheter 4.

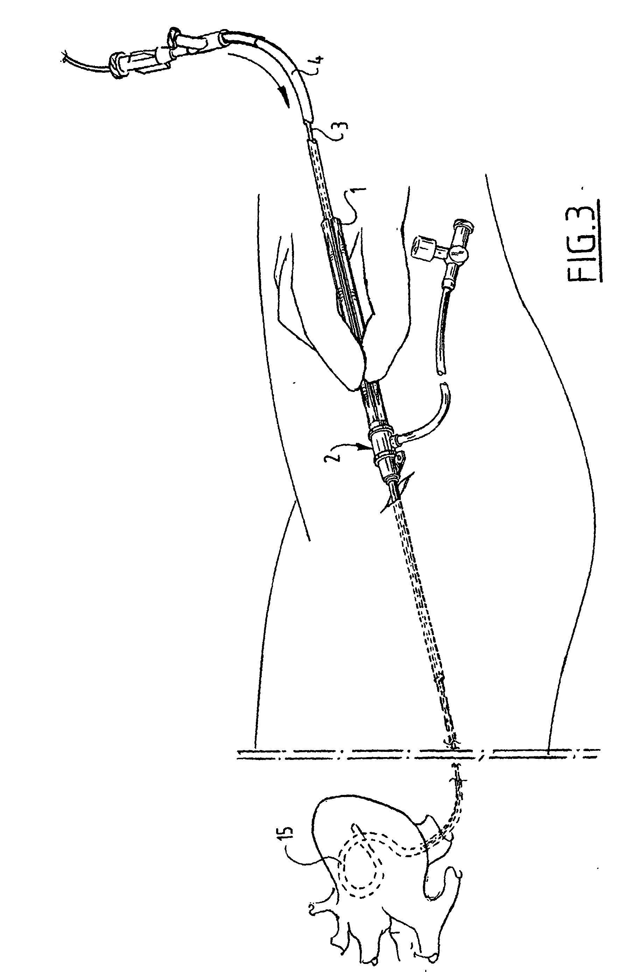

In order to properly manipulate the catheter 4 which, as has been noted, may be of the type comprising a curved tip as has been illustrated schematically in the figures 3 and 4, the tip straightener 1 is employed. The tip straightener 1 is arranged around the basic body 4 of the catheter by receiving the catheter 4 via the longitudinal slit 11 in the central channel 7 of the tip straightener 1. Subsequently the tip straightener 1 may be pushed to the tip of the catheter 4, as a result of which the tip will be straightened. When the tip straightener 1 is positioned at the tip of the catheter 4 as shown in figure 1, whereby the opening of the guide wire 5 is just within reach, the guide wire 3 can be pushed into this opening.

The body of the tip straightener 1 is in the radial direction elastically compressible. By compressing this body between thumb and index finger, the catheter 4 will be secured properly, as a result of which it can no longer move inside the tip straightener 1. Consequently it will be easy to insert the proximal end of the guide wire 3.

When the section of the guide wire 3 which is situated outside the body of the patient has been pushed through the catheter 4, the catheter may be introduced. To this end the catheter 4 is pushed by means of the tip straightener 1 over the guide wire 3 to the introduction sheath 2. As can be seen in figure 2 in particular, the relatively distal end of the tip straightener 1 has been provided with connecting means 8, which can be used to connect the tip straightener to the introduction sheath 2, so that it forms, as one whole, a continuation of the introduction sheath 2.

The tip straightener 1 also comes in useful when inserting the catheter 4 through the sealing membrane 10 of the introduction sheath 2. As the catheter 4 can be gripped tightly, sufficient axial pressure can be exerted in order to push the catheter through the membrane 10 without problems.

Next, the catheter 4 is inserted via the tip straightener 1 connected to the introduction sheath 2, as has been illustrated schematically in figure 3. As soon as the curved end 15 of the catheter 4 has been advanced to the desired position, the guide wire 3 can be removed.

The tip straightener may remain connected to the introduction sheath 2 or, as has been illustrated schematically in figure 4, be removed by undoing the connecting means 8 and removing the basic body of the catheter 4 from the tip straightener 1 via the slit 11.

Another possibility is to move the tip straightener 1 over the basic body of the catheter 4 in the proximal direction as far as the connecting member 17 thereof. In the usual manner a reinforcing sleeve 6 has been arranged at the transition from the basic body of the catheter to the connecting member 17.

The central channel 7 of the tip straightener 1 has been widened into a funnel shape at its proximal end, as can be seen at number 12. As a result the tip straightener 1 can be pushed with its proximal end onto the reinforcing sleeve 6 in such a way that it is more or less fixed to this reinforcing sleeve 6. In that case the tip straightener 1 assumes the function of the reinforcing sleeve 6 and provides at the same time a better grip on the connecting member 17.

In order to get a good grip on the tip straightener 1, the latter has been provided with a profile in the form of longitudinal ridges 16.

As can be seen in figure 2, the longitudinal slit 11 has been formed in such a way that in cross-section it has been widened towards the outside. As a result the side walls of the slit 11 form, when arranging the tip straightener 1 around the catheter, guiding means which facilitate introducing the catheter. Consequently the longitudinal slit 11 may be relatively narrow at the side of the channel 7 as a result of which the catheter 4 remains properly enclosed inside the central channel 7, also when straightening the curved tip.

With the assembly of the tip straightener 1 and the introduction sheath 2 illustrated in figure 2, connecting means acting in unison have been arranged. The connecting means 8 of the tip straightener 1 act in unison with the complementary connecting means of the introduction sheath 2.

Furthermore, the tip straightener 1 has been provided, close to the connecting means 8, with a stop in the shape of a shoulder 9 which prevents the tip straightener 1 from being pushed into the introduction sheath 2 too far, as a result of which the membrane 10 could be forced open. In this manner leakage through the membrane 10 is prevented by employing the tip straightener 1.

## Claims

1. Catheter-tip-straightener comprising a relatively stiff tubular body with a central channel (7) and a longitudinal slit (11) formed in a wall of the body for the purpose of passing a catheter (4) laterally to and from the channel, wherein on one end the body comprises connecting means (8) for the purpose of connecting it to a catheter-introduction-sheath.

2. Catheter-tip-straightener as claimed in claim 1, wherein the tubular body is elastically compressible in the radial direction.

3. Catheter-tip-straightener as claimed in claim 1 or 2, comprising a stop (9) pointing in the direction of the end supporting the connecting means.

4. Catheter-tip-straightener as claimed in claim 3, wherein the stop (9) is made up of a shoulder.

5. Catheter-tip-straightener as claimed in one of the previous claims, wherein the channel (7) has been widened into a funnel shape at the end opposite to the connecting means.

6. Catheter-tip-straightener as claimed in one of the previous claims, wherein the longitudinal slit (11) has been widened in cross-section radially towards the outside.

7. Catheter-tip-straightener as claimed in one of the previous claims, wherein the outer surface has been profiled.

8. Catheter-tip-straightener as claimed in claim 7, wherein the profile comprises longitudinal ridges.

9. Assembly of a catheter-introduction-sheath and a catheter-tip-straightener as claimed in one of the previous claims, wherein the introduction sheath (2) has been provided with connecting means acting in unison with the connecting means of the tip straightener.

## Patentansprüche

1. Kathederspitzen-Richter mit einem relativ steifen rohrförmigen Körper, der einen zentralen Kanal (7) aufweist und einen Längsschlitz (11) in der Wand des rohrförmigen Körpers besitzt, damit seitlich ein Katheder (4) in den Kanal eingesetzt und aus diesem entnommen werden kann, wobei ein Ende des rohrförmigen Körpers Verbindungsmittel (8) aufweist, um diesen mit einer Katheder-Einführungshülle zu verbinden.

2. Kathederspitzen-Richter nach Anspruch 1, bei welchem der rohrförmige Körper elastisch in Radialrichtung kompressibel ist.

3. Kathederspitzen-Richter nach den Ansprüchen 1 oder 2, welcher einen Anschlag (9) aufweist, der in Richtung des Endes weist, das die Verbindungsmittel trägt.

4. Kathederspitzen-Richter nach Anspruch 3, bei welchem der Anschlag (9) als Schulter ausgebildet ist.

5. Kathederspitzen-Richter nach einem der vorhergehenden Ansprüche, bei welchem der Kanal (7) trichterartig an jenem Ende aufgeweitet ist, das den Verbindungsmitteln entgegengesetzt ist.

6. Kathederspitzen-Richter nach einem der vorhergehenden Ansprüche, bei welchem der Längsschlitz (11) im Querschnitt radial nach der Außenseite hin aufgeweitet ist.

7. Kathederspitzen-Richter nach einem der vorhergehenden Ansprüche, bei welchem die Außen-Oberfläche profiliert ausgebildet ist.

8. Kathederspitzen-Richter nach Anspruch 7, bei welchem das Profil Längsrippen aufweist.

9. Aufbau bestehend aus einer Katheder-Einführungshülse und einem Kathederspitzen-Richter nach einem der vorhergehenden Ansprüche, wobei die Einführungshülse (2) mit Verbindungsmitteln versehen ist, die gemeinsam mit den Verbindungsmitteln des Spitzen-Richters wirken.

## Revendications

1. Redresseur de pointe de cathéter comprenant un corps tubulaire relativement rigide avec un canal central (7) et une fente longitudinale (11) formée dans une paroi du corps dans le but de faire passer un cathéter (4) latéralement vers le canal et depuis celui-ci, dans lequel une première extrémité du corps comprend des moyens de liaison (8) destinés à le relier à une gaine d'introduction de cathéter.

2. Redresseur de pointe de cathéter selon la revendication 1, dans lequel le corps tubulaire peut être compressé de manière élastique dans la direction radiale.

3. Redresseur de pointe de cathéter selon la revendication 1 ou 2, comprenant une butée (9) orientée dans la direction de l'extrémité supportant le moyen de liaison.

4. Redresseur de pointe de cathéter selon la revendication 3, dans lequel la butée (9) est constituée par un épaulement.

5. Redresseur de pointe de cathéter selon l'une quelconque des revendications précédentes, dans lequel le canal (7) est élargi en forme d'entonnoir à l'extrémité opposée par rapport au moyen de liaison.

6. Redresseur de pointe de cathéter selon l'une quelconque des revendications précédentes, dans lequel la fente longitudinale (11) est élargie en section transversale radialement vers l'extérieur.

7. Redresseur de pointe de cathéter selon l'une quelconque des revendications précédentes, dans lequel la surface externe est profilée.

8. Redresseur de pointe de cathéter selon la revendication 7, dans lequel le profil comprend des nervures longitudinales.

9. Ensemble d'une gaine d'introduction de cathéter et d'un redresseur de pointe de cathéter selon l'une des revendications précédentes, dans lequel la gaine d'introduction (2) comporte des moyens de liaison coopérant avec les moyens de liaison du redresseur de pointe.
